(19) Europäisches Patentamt
European Patent Office
Office européen des brevets

(11) Veröffentlichungsnummer: **0 019 156**
**A1**

# (12) EUROPÄISCHE PATENTANMELDUNG

(21) Anmeldenummer: **80102376.3**

(22) Anmeldetag: **02.05.80**

(51) Int. Cl.[3]: **C 07 C 127/22**
A 01 N 47/34

(30) Priorität: **12.05.79 DE 2919292**

(43) Veröffentlichungstag der Anmeldung:
**26.11.80 Patentblatt 80/24**

(84) Benannte Vertragsstaaten:
**BE CH DE FR GB IT LI NL**

(71) Anmelder: **BAYER AG**
**Zentralbereich Patente, Marken und Lizenzen**
**D-5090 Leverkusen 1, Bayerwerk(DE)**

(72) Erfinder: **Fest, Christa, Dr.**
**Im Johannistal 20**
**D-5600 Wuppertal(DE)**

(72) Erfinder: **Kraus, Peter, Dr.**
**Düsseldorfer Strasse 43**
**D-5000 Koeln 80(DE)**

(72) Erfinder: **Scheinpflug, Hans, Dr.**
**Am Thelenhof 15**
**D-5090 Leverkusen(DE)**

(54) **Substituierte Harnstoffe, Verfahren zu ihrer Herstellung und ihre Verwendung als Pflanzenbakterizide.**

(57) Die substituierten Harnstoffe der Formel

$$R^1\text{-NH-CO-}\underset{\substack{|\\R^2}}{N}\text{-CO-}R^3 \qquad (I)$$

in welcher
$R^1$ für Cycloalkyl steht,
$R^2$ für Cycloalkyl oder für gegebenenfalls substituiertes Phenyl steht und
$R^3$ für Cycloalkyl oder für substituiertes Phenyl steht.
werden erhalten, wenn man Carbodiimide mit Carbonsäuren gegebenenfalls in Gegenwart eines tertiären Amins und gegebenenfalls unter Verwendung eines Verdünnungsmittels umsetzt.

Die Wirkstoffe sind für den Gebrauch als Pflanzenschutzmittel geeignet und zeigen insbesondere eine gute Wirkung gegen Xanthomonas oryzae an Reis.

EP 0 019 156 A1

Croydon Printing Company Ltd.

BAYER AKTIENGESELLSCHAFT
Zentralbereich
Patente, Marken und Lizenzen

5090 Leverkusen, Bayerwerk
Slr/Th/Kü
Ia

# Substituierte Harnstoffe, Verfahren zu ihrer Herstellung und ihre Verwendung als Pflanzenbakterizide

Die Erfindung betrifft neue substituierte Harnstoffe, ein Verfahren zu ihrer Herstellung und ihre Verwendung als Pflanzenbakterizide.

Es ist bekannt, daß bestimmte Kupferverbindungen, wie z.B. Kupferoxychlorid, fungizide und bakterizide Eigenschaften aufweisen. Deren Wirkung ist jedoch, insbesondere bei niedrigen Wirkstoffkonzentrationen und Aufwandmengen, nicht immer zufriedenstellend.

Es wurden nun neue substituierte Harnstoffe der Formel

$$R^1\text{-NH-CO-}\underset{\displaystyle R^2}{\text{N}}\text{-CO-}R^3 \qquad (I)$$

gefunden, in welcher

$R^1$ für Cycloalkyl steht,

$R^2$ für Cycloalkyl oder für gegebenenfalls substituiertes Phenyl steht und

$R^3$ für Cycloalkyl oder für substituiertes Phenyl steht.

BAD ORIGINAL

Man erhält die substituierten Harnstoffe der Formel (I), wenn man Carbodiimide der Formel

$$R^1\text{-N=C=N-}R^2 \qquad \text{(II)}$$

in welcher

$R^1$ und $R^2$ die oben angegebenen Bedeutungen haben,

mit Carbonsäuren der Formel

$$R^3\text{-CO-OH} \qquad \text{(III)}$$

in welcher

$R^3$ die oben angegebene Bedeutung hat,

gegebenenfalls in Gegenwart eines tertiären Amins und gegebenenfalls unter Verwendung eines Verdünnungsmittels, umsetzt.

Die neuen Verbindungen der Formel (I) zeichnen sich durch hohe Wirksamkeit gegen pflanzenschädigende Bakterien aus und sind daher als Pflanzenschutzmittel von Interesse.

Überraschenderweise zeigen die erfindungsgemäßen substituierten Harnstoffe (I) eine erheblich höhere bakterizide Wirkung als aus dem Stand der Technik bekannte Verbindungen gleicher Wirkungsrichtung, wie z.B. Kupferoxychlorid.

Die Erfindung betrifft insbesondere Verbindungen der Formel (I), in welcher

$R^1$ für Cyclohexyl steht,

$R^2$ für Cyclohexyl oder für 2,6-Dialkyl-phenyl mit 1 bis 4 Kohlenstoffatomen je Alkylrest steht, und

$R^3$ für Cyclohexyl oder für Phenyl steht, welches durch $C_1$-$C_4$-Alkyl, $C_1$-$C_4$-Alkoxy, Halogen, $C_1$-$C_2$-Halogenalkyl und/oder Nitro substituiert ist.

Verwendet man als Ausgangsstoffe beispielsweise Dicyclohexyl-carbodiimid und 4-Methoxy-benzoesäure, so kann die Reaktion dieser Verbindungen durch folgendes Formelschema skizziert werden:

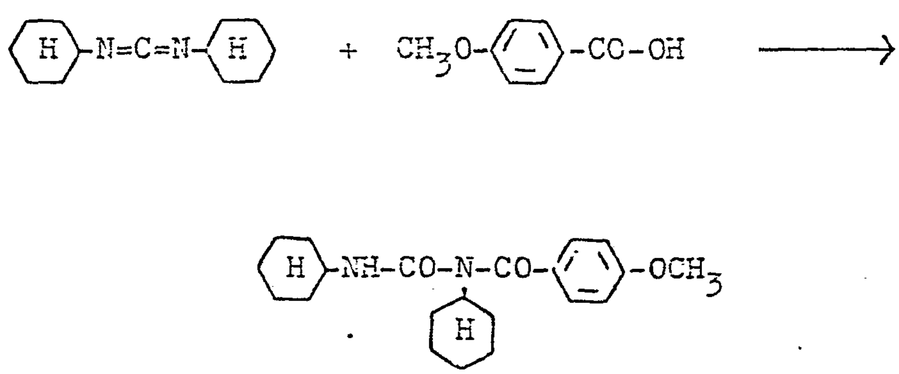

Die als Ausgangsstoffe zu verwendenden Carbodiimide sind durch Formel (II) definiert. Vorzugsweise stehen darin $R^1$ und $R^2$ für diejenigen Reste, welche bei der Definition der Reste $R^1$ und $R^2$ in Formel (I) als bevorzugt genannt sind.

Als Beispiele seien genannt:
Dicyclohexyl-carbodiimid, N-(2,6-Dimethyl-phenyl)-,

N-(2,6-Diäthyl-phenyl)-, N-(2-Methyl-6-äthyl-phenyl)-, N-(2,6-Di-n-propyl-phenyl)-, N-(2,6-Di-iso-propyl-phenyl)-, N-(2,6-Di-n-butyl-phenyl)- und N-(2,6-Di-isobutyl-phenyl)- -N'-cyclohexyl-carbodiimid.

Die Carbodiimide der Formel (II) sind bekannte Verbindungen (vergleiche Methodicum Chimicum, Band 6 (1974), S. 783-794, Georg-Thieme-Verlag Stuttgart und Academic Press New York, San Francisco, London). Bekannt ist auch deren Herstellung aus Harnstoffen oder Thioharnstoffen durch Abspaltung von Wasser bzw. Schwefelwasserstoff. Als Kondensationsmittel kann Toluolsulfonsäurechlorid dienen, Schwefelwasserstoff kann mit Bleicarbonat abgespalten werden. Weiterhin können durch Reaktion von Cyclohexylisocyanat mit Aminen bzw. Anilinen unter Abspaltung von Wasser die Carbodiimide erhalten werden.

Die weiter als Ausgangsstoffe zu verwendenden Carbonsäuren sind durch Formel (III) definiert. Vorzugsweise hat darin $R^3$ die bei der Restedefinition für Formel (I) als bevorzugt angegebene Bedeutung.

Als Beispiele für die Carbonsäuren der Formel (III) seien genannt:
Cyclohexancarbonsäure, 4-Methyl-, 3-Methyl-, 2-Methyl-, 4-Iso-propyl-, 4-tert.-Butyl-, 4-Methoxy-, 3-Methoxy-, 2-Chlor-, 3-Chlor-, 4-Chlor-, 2,4-Dichlor-, 2,5-Dichlor-, 3,4-Dichlor-, 2,6-Dichlor-, 2-Brom-, 3-Brom-, 4-Brom-, 3-Fluor-, 4-Fluor-, 2-Chlor-5-brom-, 3-Trifluormethyl-, 2-Nitro-, 3-Nitro-, 4-Nitro-, 5-Chlor-2-nitro-, 4-Chlor-3-nitro-, 2-Methyl-3-nitro-, 3-Methyl-2-nitro-, 3-Methyl-4-nitro-, 4-Methyl-3-nitro- und 5-Methyl-2-nitro-benzoesäure.

Die Carbonsäuren der Formel (III) sind allgemein bekannte, laboratoriumsübliche Verbindungen.

Das Verfahren zur Herstellung der erfindungsgemäßen substituierten Harnstoffe der Formel (I) wird bevorzugt unter Verwendung geeigneter Lösungs- oder Verdünnungsmittel durchgeführt. Als solche kommen praktisch alle inerten organischen Solventien infrage. Hierzu gehören insbesondere aliphatische und aromatische, gegebenenfalls chlorierte Kohlenwasserstoffe, wie Benzin, Benzol, Toluol, Xylol, Methylenchlorid, Chloroform, Tetrachlorkohlenstoff, Chlorbenzol und o-Dichlorbenzol, Äther wie Diäthyl- und Dibutyläther, Tetrahydrofuran und Dioxan, Ketone wie Aceton, Methyläthyl-, Methylisopropyl- und Methylisobutylketon, Nitrile wie Acetonitril und Propionitril sowie Alkohole wie Methanol, Äthanol, n- und iso-Propanol.

Tertiäre Amine, welche beim erfindungsgemäßen Verfahren als Katalysatoren eingesetzt werden können, sind beispielsweise Trimethylamin, Triäthylamin, Äthyl-di-isopropylamin, Äthyl-dicyclohexylamin, N,N-Dimethylanilin, N,N-Dimethyl-benzylamin und Diaza-bicyclononan.

Die Reaktionstemperatur kann innerhalb eines größeren Bereichs variiert werden. Im allgemeinen arbeitet man zwischen 0 und 15o°C, vorzugsweise bei 2o bis 1oo°C.

Das erfindungsgemäße Verfahren wird im allgemeinen bei Normaldruck durchgeführt.

Zur Durchführung des erfindungsgemäßen Verfahrens werden die Ausgangsstoffe gewöhnlich in äquimolaren Mengen eingesetzt. Ein Überschuß der einen oder anderen Reaktionskomponente bringt keine wesentlichen Vorteile. Die Umsetzung wird im allgemeinen in einem geeigneten Verdünnungsmittel in Gegenwart eines tertiären Amins durchgeführt und das Reaktionsgemisch wird mehrere Stunden bei der erforderlichen Temperatur gerührt. Nach Abdestillieren des Lösungsmittels im Vakuum erhält man die Produkte im allgemeinen in kristalliner Form. Zur Charakterisierung der Produkte, welche gewöhnlich durch Umkristallisation gereinigt werden, dient der Schmelzpunkt.

Die erfindungsgemäßen Wirkstoffe weisen eine starke mikrobizide Wirkung auf und können zur Bekämpfung von unerwünschten Mikroorganismen praktisch eingesetzt werden. Die Wirkstoffe sind für den Gebrauch als Pflanzenschutzmittel geeignet und können als Bakterizide eingesetzt werden.

Bakterizide Mittel werden im Pflanzenschutz zur Bekämpfung von Pseudomonadaceae, Rhizobiaceae, Enterobacteriaceae, Corynebacteriaceae und Streptomycetaceae eingesetzt.

So zeigen die erfindungsgemäßen Wirkstoffe eine Wirkung gegen Xanthomonas oryzae an Reis. Neben einer Wirkung nach Applikation auf das Blatt haben die neuen Verbindungen auch eine deutliche systemische Wirkung, die nach Applikation zum Bewässerungswasser oder auf den Boden erkennbar wird.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, des Bodens und des Bewässerungswassers.

Die Wirkstoffe können in die üblichen Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä., sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chloräthylene oder Methylenchlorid, aliphatische Kohlenwasserstoffe, wie Cyclohexan oder Paraffine, z.B. Erdölfraktionen, Alkohole, wie Butanol oder Glycol sowie deren Äther und Ester, Ketone, wie Aceton, Methyläthylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgas, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen in Frage: z.B. nichtionogene und anionische Emulgatoren, wie Polyoxyäthylen-Fettsäure-Ester, Polyoxyäthylen-Fettalkohol-Äther, z.B. Alkylarylpolyglykol-äther, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat.

Es können Farbstoffe wie anorganische Pigmente, z.B. Eisenoxid, Titanoxid, Ferrocyanblau und organische Farbstoffe, wie Alizarin-, Azol-Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in den Formulierungen oder in den verschiedenen Anwendungsformen in Mischung mit anderen bekannten Wirkstoffen vorliegen, wie Fungiziden, Bakteriziden, Insektiziden, Akariziden, Nematiziden, Herbiziden, Schutzstoffen gegen Vogelfraß, Wuchsstoffen, Pflanzennährstoffen und Bodenstrukturverbesserungsmitteln.

Die Wirkstoffe können als solche, in Form ihrer Formulierungen oder der daraus durch weiteres Verdünnen bereiteten Anwendungsformen, wie gebrauchsfertige Lösungen, Emulsionen, Suspensionen, Pulver, Pasten und Granulate angewendet werden. Die Anwendung geschieht in üblicher Weise, z.B. durch Gießen, Tauchen, Spritzen, Sprühen, Vernebeln, Verdampfen, Injizieren, Verschlämmen, Verstreichen, Stäuben, Streuen, Trockenbeizen, Feuchtbeizen, Naßbeizen, Schlämmbeizen oder Inkrustieren.

Bei der Behandlung von Pflanzenteilen können die Wirkstoffkonzentrationen in den Anwendungsformen in einem größeren Bereich variiert werden. Sie liegen im allgemeinen zwischen 1 und 0,0001 Gew.-%, vorzugsweise zwischen 0,5 und 0,001 %.

Bei der Saatgutbehandlung werden im allgemeinen Wirkstoffmengen von 0,001 bis 50 g je Kilogramm Saatgut, vorzugsweise 0,01 bis 10 g, benötigt.

Bei Behandlung des Bodens sind Wirkstoffkonzentrationen von 0,00001 bis 0,1 Gew.-%, vorzugsweise von 0,0001 bis 0,02 %, am Wirkungsort erforderlich.

Beispiel A

Xanthomonas oryzae-Test / Bakteriose / Reis

Zur Herstellung einer zweckmäßigen Wirkstoffzubereitung vermischt man 1 Gewichtsteil Wirkstoff mit 25 Gewichtsteilen eines Lösungsmittels (Aceton) und 0,75 Gewichtsteilen eines Dispergiermittels (Alkylarylpolyglykoläther) und verdünnt das Konzentrat mit Wasser auf die gewünschte Konzentration.

Mit der Spritzflüssigkeit bespritzt man vier Wochen alte Reispflanzen der Sorte Kinmaze bis zur Tropfnässe, läßt sie abtrocknen und inokuliert die Pflanzen mit Xanthomonas oryzae, indem man Nadeln in eine wäßrige Bakteriensuspension taucht und die Blätter durch Stiche verletzt.

Nach einer 48-stündigen Inkubation bei 100 % rel. Leuftfeuchtigkeit verbleiben die Pflanzen 10 Tage bis zur Auswertung in einem Gewächshaus bei 24 bis 26°C und 70 bis 80 % rel. Luftfeuchtigkeit. Der Befallsgrad wird in Prozent des Befalls der unbehandelten Kontrollpflanzen ausgedrückt: 0 % bedeutet keinen Befall, 100 % entsprechen dem Befall der Kontrollpflanzen.

Bei diesem Test zeigen z.B. die folgenden Verbindungen eine überlegene Wirkung gegenüber dem Stand der Technik:

Verbindungen gemäß Herstellungsbeispielen 6 und 7.

Beispiel B

Xanthomonas oryzae-Test / Bakteriose / Reis / systemisch

Zur Herstellung eines zweckmäßigen Wirkstoffzubereitung vermischt man 1 Gewichtsteil Wirkstoff mit 125 Gewichtsteilen eines Lösungsmittels (Aceton) und 3,75 Gewichtsteilen eines Dispergiermittels (Alkylarylpolyglykolether) und verdünnt das Konzentrat mit Wasser auf die gewünschte Konzentration.

10 x 10 cm große Töpfe, in denen ca. 5 Wochen alte Reispflanzen der Sorte Kinmaze stehen, werden mit einer definierten Menge dieser Wirkstoffzubereitung gegossen, wobei sich die Aufwandmenge des Wirkstoffes aus der Bodenfläche ergibt.

3 Tage nach der Behandlung des Bodens werden die Pflanzen mit Xanthomonas oryzae inokuliert, indem man Nadeln in eine wäßrige Bakteriensuspension taucht und die Blätter durch Stiche verletzt. Bis zur Auswertung verbleiben die Pflanzen in einem Gewächshaus bei 24 bis 26°C und 70 bis 80 % rel. Luftfeuchtigkeit.

Der Befallsgrad wird in Prozent des Befalls der unbehandelten Kontrollpflanzen ausgedrückt: 0 % bedeutet keinen Befall, 100 % entsprechen dem Befall der Kontrollpflanzen.

Bei diesem Test zeigen die folgenden Verbindungen eine überlegene Wirkung gegenüber dem Stand der Technik:

Verbindungen gemäß Herstellungsbeispielen 1, 4, 7, 8 und 9.

## Herstellungsbeispiele

### Beispiel 1

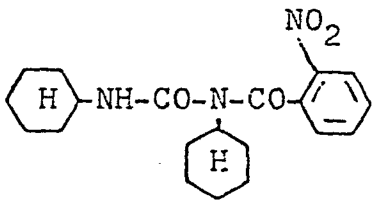

Eine Lösung von 25 g (o,15 Mol) 2-Nitro-benzoesäure in 75 ml Methanol wird zu einer siedenden Lösung von 31 g (o,15 Mol) Dicyclohexyl-carbodiimid und 8 ml Triäthylamin in 3oo ml Methanol tropfenweise gegeben. Das Reaktionsgemisch wird 2 Stunden unter Rückfluß erhitzt und über Nacht bei Raumtemperatur gerührt. Nach Abdestillieren des Lösungsmittels im Vakuum wird der Rückstand aus Isopropanol umkristallisiert. Man erhält 25 g (45% der Theorie) N-(2-Nitro-benzoyl)-N,N'-dicyclohexyl-harnstoff vom Schmelzpunkt 134°C.

Analog Beispiel 1 können die folgenden Verbindungen der Formel (I a) hergestellt werden:

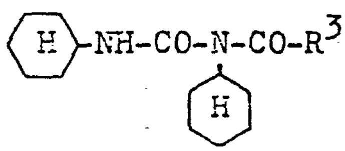

(I a)

| Beispiel Nr. | $R^3$ | Schmelzpunkt (°C) | Ausbeute (% der Theorie) |
|---|---|---|---|
| 2 | 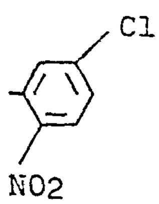 | 166 | 33 |

| Beispiel Nr. | $R^3$ | Schmelzpunkt (oC) | Ausbeute (% der Theorie) |
|---|---|---|---|
| 3 | 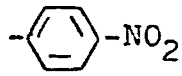 | 2o4 | 86 |
| 4 | 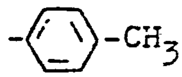 | 133 | 41 |
| 5 | 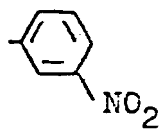 | 175 | 56 |
| 6 | 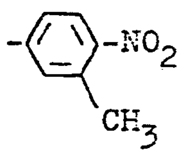 | 19o | 55 |
| 7 | 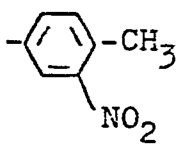 | 175 | 52 |
| 8 | 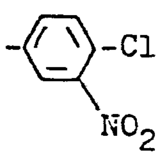 | 18o | 47 |
| 9 | 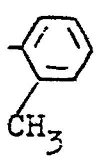 | 143 | 7o |

| Beispiel Nr. | $R^3$ | Schmelzpunkt (°C) | Ausbeute (% der Theorie) |
|---|---|---|---|
| 1o | 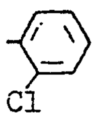 | 167 | 63 |
| 11 | 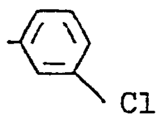 | 175 | 5o |
| 12 | 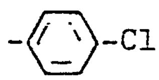 | 175 | 55 |
| 13 | 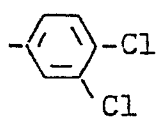 | 189 | 68 |
| 14 | 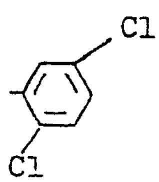 | 177 | 43 |
| 15 | 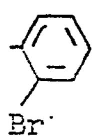 | 154 | 38 |
| 16 | 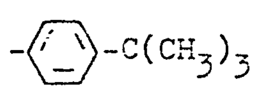 | 162 | 42 |
| 17 | 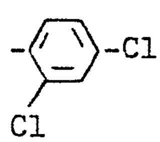 | 154 | 59 |

| Beispiel Nr. | $R^3$ | Schmelzpunkt (°C) | Ausbeute (% der Theorie) |
|---|---|---|---|
| 18 | 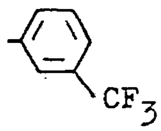 | 165 | 4o |
| 19 | 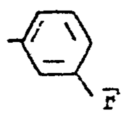 | 165 | 58 |
| 2o | 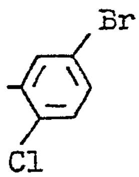 | 177 | 47 |
| 21 | 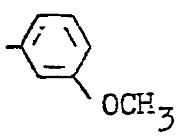 | 145 | 43 |
| 22 | 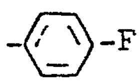 | 178 | 48 |
| 23 | 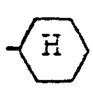 | 148 | 62 |
| 24 | 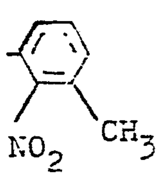 | 158-62 | 65 |

| Beispiel Nr. | $R^3$ | Schmelzpunkt (°C) | Ausbeute (% der Theorie) |
|---|---|---|---|
| 25 | 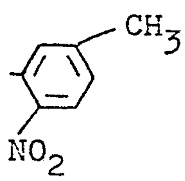 | 176-80 | 53 |
| 26 | 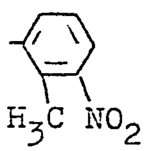 | 176-81 | 57 |

Beispiel 27 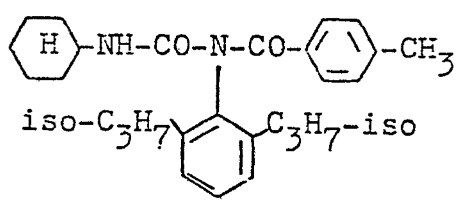

Die Verbindung wird analog Beispiel 1 in einer Ausbeute von 17 % der Theorie erhalten. Der Fp. beträgt 238°C.

Patentansprüche

1. Substituierte Harnstoffe der allgemeinen Formel

$$R^1\text{-NH-CO-}\underset{\substack{|\\R^2}}{\text{N}}\text{-CO-}R^3$$

in welcher

$R^1$ für Cycloalkyl steht,
$R^2$ für Cycloalkyl oder für gegebenenfalls substituiertes Phenyl steht und
$R^3$ für Cycloalkyl oder für substituiertes Phenyl steht.

2. Verfahren zur Herstellung von substituierten Harnstoffen der allgemeinen Formel I, dadurch gekennzeichnet, daß man Carbodiimide der Formel

$$R^1\text{-N=C=N-}R^2 \qquad \text{(II)}$$

in welcher
$R^1$ und $R^2$ die in Anspruch 1 angegebenen Bedeutungen haben,
mit Carbonsäuren der Formel

$$R^3\text{-CO-OH} \qquad \text{(III)}$$

in welcher
$R^3$ die in Anspruch 1 angegebene Bedeutung hat,

gegebenenfalls in Gegenwart eines tertiären Amins und gegebenenfalls unter Verwendung eines Verdünnungsmittels, umsetzt.

3. Bakterizide Mittel, gekennzeichnet durch einen Gehalt an mindestens einem substituierten Harnstoff der Formel I.

4. Verfahren zur Bekämpfung von Bakterien, dadurch gekennzeichnet, daß man substituierte Harnstoffe der Formel I auf Bakterien oder ihren Lebensraum einwirken läßt.

5. Verwendung von substituierten Harnstoffen der Formel I zur Bekämpfung von Bakterien.

6. Verfahren zur Herstellung von bakteriziden Mitteln, dadurch gekennzeichnet, daß man substituierte Harnstoffe der Formel I mit Streckmitteln und/oder oberflächenaktiven Mitteln vermischt.

Europäisches Patentamt

# EUROPÄISCHER RECHERCHENBERICHT

Nummer der Anmeldung: EP 80 10 2376

## EINSCHLÄGIGE DOKUMENTE

| Kategorie | Kennzeichnung des Dokuments mit Angabe, soweit erforderlich, der maßgeblichen Teile | betrifft Anspruch | KLASSIFIKATION DER ANMELDUNG (Int Cl. 3) |
|---|---|---|---|
| X | JOURNAL OF THE CHEMICAL SOCIETY, November 1964<br>LONDON (GB)<br>N. PRAVDIC et al.: "Glucuronic Esters. Part I. Synthesis of Methyl 2,3,4-Tri-O-acetyl-I-O-acyl-D-glucopyranuronates by Use of Carbodi-imide", Seiten 4633-4635.<br>* Tabelle 2, Seite 4635 *<br>----- | 1,2 | C 07 C 127/22<br>A 01 N 47/34 |

RECHERCHIERTE SACHGEBIETE (Int. Cl. 3)

C 07 C 127/22
A 01 N 47/34

KATEGORIE DER GENANNTEN DOKUMENTE

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

X Der vorliegende Recherchenbericht wurde für alle Patentansprüche erstellt.

| Recherchenort | Abschlußdatum der Recherche | Prüfer |
|---|---|---|
| Den Haag | 18-08-1980 | GAUTIER |